# EUROPÄISCHE PATENTSCHRIFT

(11) **EP 0 872 223 B1**
(45) Veröffentlichungstag und Bekanntmachung des Hinweises auf die Patenterteilung: **26.03.2003**
(21) Anmeldenummer: 97810235.8
(22) Anmeldetag: 16.04.1997
(51) Int. Cl.: A61F 2/46, B05C 17/005

(54) **Einfüllvorrichtung für Knochenzement**
Filling apparatus for bone cement
Dispositif de remplissage pour ciment à os

(43) Veröffentlichungstag der Anmeldung: 21.10.1998
(73) Patentinhaber: Sulzer Orthopädie AG, 6340 Baar (CH)
(72) Erfinder: Heller, Mathias, 8404 Winterthur (CH); Willert, Hans-Georg, Prof. Dr.Med., 37075 Göttingen (DE)
(74) Vertreter: Manitz, Finsterwald & Partner Gbr

(56) Entgegenhaltungen:
- EP-A- 0 628 295
- GB-A- 2 105 198
- US-A- 2 836 333
- US-A- 5 322 381
- US-A- 5 468 245

## Beschreibung

Die Erfindung handelt von einer Einfüllvorrichtung für das Einbringen von Knochenzement, in den intramedullären Kanal eines Knochens, vorzugsweise in den Kanal eines Femurknochens, mit einer Einfüllkanüle, an deren Ende eine schlauchartige Manschette befestigt ist, die unter dem Druck von austretenden Knochenzement radial expandierbar ist.

Einfüllvorrichtungen sind in der EP-A-0 628 295 beschrieben. Als Manschette sind dort flexible Fortsetzungen einer Einfüllkanüle gezeigt, die zu ihrem freien Ende eine durch Ueberlappung, Elastizität oder Faltung gebildete Materialreserve aufweisen. Einfüllvorrichtungen dieser Art haben den Nachteil, dass sie beim Einfahren der Einfüllkanüle in den intramedullären Kanal mit der Manschettenkante an der Kanalwand aufstehen bevor das eigentliche Ende vom Kanal erreicht ist. Bei einer zu steifen Manschette besteht das Problem, dass ihre Elastizität nicht genügt, um während dem Füllen des intramedullären Kanals einen ausreichenden. Dichtdruck an einem sich verbreiterndem Kanalquerschnitt zu erzeugen. Es nützt auch nicht viel, wenn der Durchmesser von Einfüllkanüle und Manschette besonders klein gewählt werden, da dadurch neue Probleme entstehen.

Zum einen wird wegen der ständig wachsenden Viskosität vom Knochenzement das Auspressen erschwert und zum anderen werden noch höhere Anforderungen an die Manschette bezüglich Ausdehnung gestellt.

Andere Ausführungen wie die GB-A-2 105 198 setzen zusätzliche Elemente wie axial verschiebbare Schutzhülsen auf einer Einfüllkanüle voraus, deren Ende als ein aufspannbarer Schirm mit Rippen und dazwischen liegenden Folien ausgebildet ist.

Aufgabe der vorliegenden Erfindung ist es, eine einfache Einfüllvorrichtung zu schaffen, die es gestattet einen intramedullären Kanal von seinem Ende her gegen einen manuell erzeugten Gegendruck mit Knochenzement zu füllen.

Diese Aufgabe wird mit den Merkmalen von Anspruch 1 gelöst.

Eine solche Einfüllkanüle hat den Vorteil, dass sie ohne Verzögerung d.h. ohne Beanspruchung eines stark schwankenden Anteils an der Aushärtezeit von Knochenzement im Kanalgrund plaziert werden kann. Ein weiterer Vorteil ist, dass beim Einfahren in ovale Kanalquerschnitte automatisch eine Lage eingenommen wird, die beim Ausfahren unter Druck einer zu grossen lokalen Ueberdehnung der Manschette entgegenwirkt. Die Einfüllkanüle lässt sich bis in den Kanalgrund auf eine Markraumsperre absenken und erzeugt mit ihrem Aufsitzen eine grosse und spürbare Gegenkraft. Beim Absenken in den Kanalgrund verformt sich der eher ovale Querschnitt problemlos in einen runden Querschnitt durch den Seitendruck der Kanalwand. Trotzdem steht im proximalen Bereich ein eigentlich ovaler Querschnitt für die Verformung unter Druck zu Verfügung.

Vorteilhafte Weiterbildungen ergeben sich aus den abhängigen Ansprüchen 2 bis 12.

Ein Aufblasen und Anliegen der Manschette ergibt sich, wenn der untere Rand der Manschette gegenüber einer tiefsten seitlichen Umlenkkante der untersten Austrittsöffnung um einen Abstand 5 mm < a < 50 mm vorsteht, wenn der expandierbare Teil der Manschette in einem Abstand 3 mm < 1 < 50 mm über einer höchsten seitlichen Austrittskante der Einfüllkanüle befestigt ist und wenn der expandierbare Teil der Manschette aus Gummi mit einer Dicke 1 mm < s < 2 mm besteht.

Durch die-gewählten Abmessungen entsteht eine Manschette die so gut dichtet, dass der Chirurg den Druck, bei dem Einfüllkanüle und Manschette auf dem Knochenzement nach oben reiten, mit seinem Gewicht auf der Einfüllvorrichtung selber bestimmt.

Eine Einfüllkanüle mit seitlichen Ausflussöffnungen hat den Vorteil, dass die Ausflussöffnung auch beim Start voll offen ist und somit unabhängig vom Operateur gleiche Bedingungen für das Auspressen vom Knochenzement geschaffen werden. Ausserdem kann die Einrichtung nur soweit zum Kanalgrund abgesenkt werden, bis der Boden der Einfüllkanüle aufsitzt. Bei einer Shore-Härte von Shore A35 hat sich eine Dicke S von 1,5 mm für den expandierbaren Teil der Manschette bewährt, wobei für frontale Austrittsöffnungen die Abstände a = 15 mm und l = 20 mm und für seitliche Austrittsöffnungen a = 15 mm und l = 5 mm bei Femurknochen eine Manschette mit optimalem Widerstand beim Einfahren und beim Ausfahren unter Druck ergeben. Bei einer Einfüllkanüle mit rundem Querschnitt wird eine im Einspannquerschnitt runde Manschette an der Einfüllkanüle beispielsweise mit einer Klemmbride befestigt, wobei der Querschnitt sich zu einer Glocke mit elliptischem Querschnitt erweitert. In diesem wachsenden Querschnittsbereich kann die Wandstärke s auf über 2 mm vergrössert werden, um ein Umstülpen der Manschette unter Innendruck zu verhindern. Die Sicherheit gegen Hängenbleiben der Manschette beim Absenken ist durch das Einziehen der unteren Kante zusätzlich erhöht.

Die Einfüllkanüle ist mit einem Auspresszylinder verbindbar, aus dem der gemischte Knochenzement mit einem Ausstosskolben ausgepresst wird.

Mit Vorteil wird vor dem Einfahren der Manschette ein Siegel auf der Einfüllkanüle zwischen Manschette und Auspresszylinder aufgesteckt, um eine Abschlussdichtung für das spätere Auspressen von Knochenzement im proximalen Kanal zu haben und eine Orientierungshilfe für einen später abgesenkten künstlichen Femurschaft zu besitzen. Aus diesem Grund ist das Siegel transparent ausgeführt, was sein Positionieren auf einem resektierten Femurknochen erleichtert, und auf seiner Innenseite mit elastischen Lippen aus Gummi ausgestattet, die ein Herausziehen von Einfüllkanüle mit Manschette ermöglichen.

Nach dem Absenken des künstlichen Femurschaftes verbleibt das Siegel an Ort bis der Knochenzement ausgehärtet ist. Der beim Absenken der Prothese herausquellende Knochenzement wird vorher entfernt. Nach dem Aushärten kann das Siegel entfernt werden, wobei es entweder aufgeschnitten wird oder von vornherein als abnehmbarer geschlitzter Ring ausgeführt ist. Im letzteren Fall ist ein der Form angepasster Verstärkungsbügel von Vorteil.

Im folgenden ist die Erfindung anhand von Beispielen beschrieben. Es zeigen:
- Fig.1: Schematisch einen Längsschnitt durch eine Einfüllkanüle mit einer erfindungsgemässen Manschette;
- Fig. 2: schematisch einen um 90° versetzten Längsschnitt durch die Manschette von Fig. 1;
- Fig. 3: schematisch eine Draufsicht auf die Einfüllkanüle und Manschette von Fig. 1;
- Fig. 4: schematisch einen Längsschnitt wie in Fig. 2 jedoch mit einer Einfüllkanüle mit seitlichen Ausflussöffnungen;
- Fig. 5: schematisch einen Ausschnitt aus einem Röhrenknochen mit Markraumsperre und einer im Aufsteigen begriffenen Einfüllkanüle mit Manschette nach Fig. 1;
- Fig. 6: schematisch eine Draufsicht auf ein Siegel, das als geschlitzter Ring mit Drahtbügel ausgeführt ist;
- Fig. 7: schematisch als Explosionszeichnung die Ansicht einer Einfüllvorrichtung für Knochenzement;
- Fig. 8a,b,c,d,e,f:: Schematisch den Ablauf für das Einfüllen von Knochenzement und das Einsetzen von einem Prothesenschaft;
- Fig. 9: schematisch eine Ansicht mit Teilschnitt durch das Siegel von Fig. 6; und
- Fig. 10: schematisch einen Schnitt durch das Siegel von Fig. 6.

In den Figuren ist eine Einfüllvorrichtung für das Einbringen von Knochenzement in den intramedullären Kanal eines Knochens, vorzugsweise in den Kanal eines Femurknochens gezeigt. Die Vorrichtung besitzt eine Einfüllkanüle 2, an deren Ende eine Manschette 3 aus Gummi in Blechglockenform 9 angebracht ist, welche mit ihrem beweglichen Teil 7 radial über den Durchmesser und axial über eine unterste Austrittsöffnung der Einfüllkanüle 2 übersteht. Dadurch, dass die Manschette 3 im expandierbaren unteren Teil mit einer Dicke 1 mm < s < 2 mm ausgeführt ist und oberhalb einer höchsten Austrittsöffnung 13 der Einfüllkanüle in einem Abstand 3 mm < l < 50 mm befestigt ist und über eine tiefste seitliche Umlenkkante 11a einer untersten Austrittsöffnung um einen Abstand 5 mm < a < 50 mm mit ihrer Unterkante 16 nach unten vorsteht, passt sie sich beim Einbringen ohne Hängenbleiben mit ihrem Querschnitt der Kanalwand an und dichtet sie beim Auspressen vom Knochenzement ab.

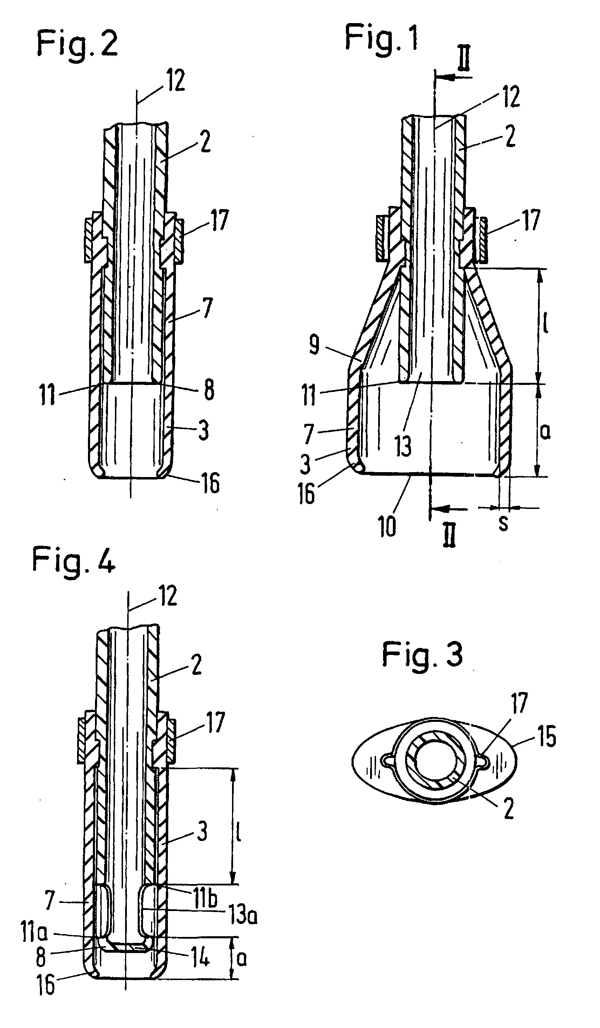

In den Figuren 1 bis 3 ist der bewegliche Teil 7 der Manschette 3 in einem Abstand 3 mm < l < 50 mm über der seitlichen Umlenkkante 11 der höchsten Austrittsöffnung 13 mit einer Klemmbride 17 befestigt. Der bewegliche Teil 7 besteht aus Gummi, hat einen elliptischen Querschnitt 15, eine Wandstärke 1 mm < s < 2 mm und die Form einer Blechglocke 9. An ihrer unteren Kante 16 ist die Manschette 3 nach innen eingezogen und steht um einen Abstand 5 mm < a < 50 mm über die tiefste seitliche Umlenkkante 11a der Austrittsöffnung 13 vor. Die Austrittsöffnung 13 liegt senkrecht zur Achse 12 der Einfüllkanüle an deren Ende 8.

In Fig. 4 ist als weiteres Beispiel eine Einfüllkanüle 2 mit seitlichen Austrittsöffnungen 13a und einem Boden 14 an deren Ende 8 gezeigt. Die Manschette 3 entspricht derjenigen von Fig. 1 bis 3 und erweitert sich ebenfalls von ihrer Einspannstelle 17 zu einem beweglichen Teil 7 mit einer Blechglockenform 9 mit elliptischem Querschnitt. Durch die über den Boden 14 und über die tiefste seitliche Umlenkkante 11a der Austrittsöffnungen 13a vorstehende Kante 16 ist selbst bei unsanftem Aufsetzen der Einfüllkanüle 2 auf dem Kanalgrund ein Aufspreizen und Dichten der Manschette 3 gegen die Kanalwand gesichert, um über eine Last in der Richtung der Achse 12 der Einfüllkanüle einen passenden Gegendruck für den austretenden Knochenzement aufzubauen. Die Einspannstelle 17 ist von um den Abstand 1 von einer höchsten seitlichen Umlenkkante 11b der Austrittsbohrungen 13a entfernt.

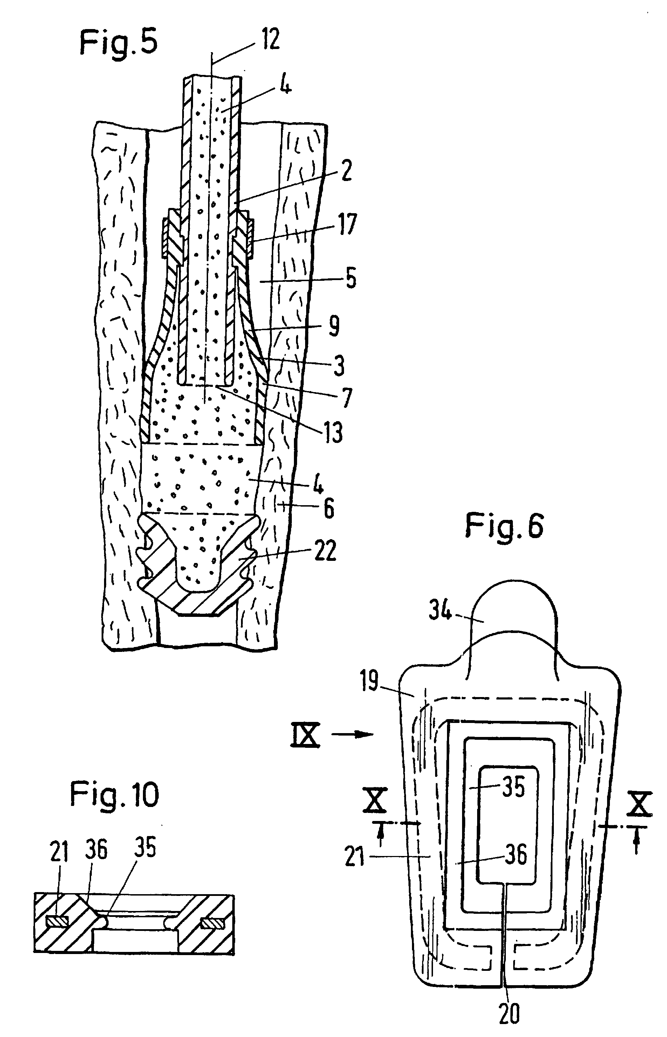

In Fig. 5 ist ein Femurknochen 6 durch eine Markraumsperre 22 verschlossen. Knochenzement 4 wird aus der Austrittsöffnung 13 ausgepresst und hat bereits den Raum zwischen Manschette 3 und Markraumsperre 22 ausgefüllt. Beim Auftreffen des expandierbaren Teils 7 der Manschette auf der Markraumsperre 22 war noch ein Luftpolster vorhanden. Dieses ist durch den austretenden Knochenzement verdrängt worden und zwischen Kanalwand 5 und Manschette 3 nach oben ausgetreten. Es ist denkbar kurz vor der Klemmstelle 17 im beweglichen Teil 7 der Manschette oder über die Klemmstelle eine kleine Entlastungsöffnung anzubringen, aus der Luft gerade noch entweichen kann. Die Einfüllkanüle wird dann in ihrer untersten Stellung gehalten bis vorhandene Luft verdrängt ist. Der nachfolgende Knochenzement spannt die Manschette 3 gegen die Kanalwand 5 und stösst sie gleichzeitig entsprechend der Auspressmenge des Knochenzements nach oben. Der Operateur spürt die Hebebewegung und kann manuell einen entsprechenden Gegendruck geben, um den Knochenzement während seines Ablegens in Vertiefungen der Kanalwand 5 zu zwingen.

In den Figuren 6, 9 und 10 ist ein Siegel 19 aus Silikongummi gezeigt, welches auf die Einfüllkanüle 2 aufsteckbar ist, um im proximalen Bereich eines Femurknochens 6 eine Dichtwirkung zu erreichen und den Knochenzement bis an das Ende des resektierten Knochens unter Druck auspressen zu können. Das Siegel 19 ist als geschlitzter Ring 20 ausgeführt, in dem ein Drahtbügel zur Versteifung eingelegt ist. Die mittlere Oeffnung ist durch eine elastische Lippe begrenzt, durch welche die Manschette 3 unter Seitendruck ausziehbar ist. Die Dimensionen der Lippe und der Manschette sind so auf den proximalen Teil einer später einzusetzenden Prothesenschaftes abgestimmt, dass die Lippe gleichzeitig auch Führung für den proximalen Teil der Prothese gibt. Zur Lippe 35 besteht ein Einlauf in Form einer Anschrägung 35, die bei der zentrierung des Prothesenschaftes hilft und auf der sich beim Einfahren des Prothesenschaftes überflüssiger Knochenzement zum Abstreifen sammelt. Der elastische Teil des Siegels 19 besteht aus transparentem Material, um die Lage des Siegels beim Positionieren auf der Resektionsfläche kontrollieren zu können. Am Siegel 19 ist ein seitlicher Haltegriff 34 angeformt, der in der Fortsetzung des grossen Trochanters zu liegen kommt.

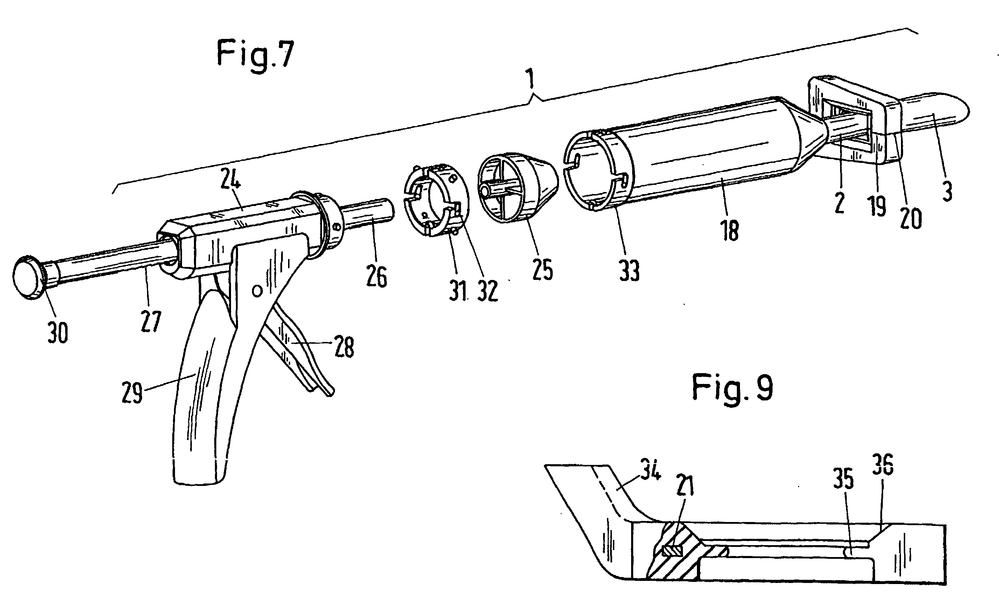

In Fig. 7 sind die Hauptkomponenten einer Einfüllvorrichtung 1 aufgeführt. Die Einfüllkanüle 2 ist mit einem Auspresszylinder 18 verbunden. Zwischen Manschette 3 und Auspresszylinder 18 ist ein Siegel 19 in Form eines geschlitzten Ringes 20 aufgesteckt. Knochenzement (hier nicht gezeigt) wird in gemischter Form in den Auspresszylinder 18 gebracht oder im Auspresszylinder gemischt. Ein Kolben 25 schliesst den Auspresszylinder ab. Eine Kupplung 31 sichert mit Bajonettverschluss 33 den Kolben 25. Ueber einen weiteren Bajonettverschluss 32 wird eine Vorschubeinrichtung 24 angeflanscht. Eine mit einem Rückholknopf 30 in ihre hintere Stellung gebrachte Schubstange 26 ist mit einer Rasterung 27 versehen, welche über einen Hebel 28 und eine nicht gezeigte Klinke eine schrittweise Vorschubbewegung erlaubt. Die ganze Einfüllvorrichtung kann an einem Pistolengriff 29 gehalten werden und der Vorschub einhändig betätigt werden.

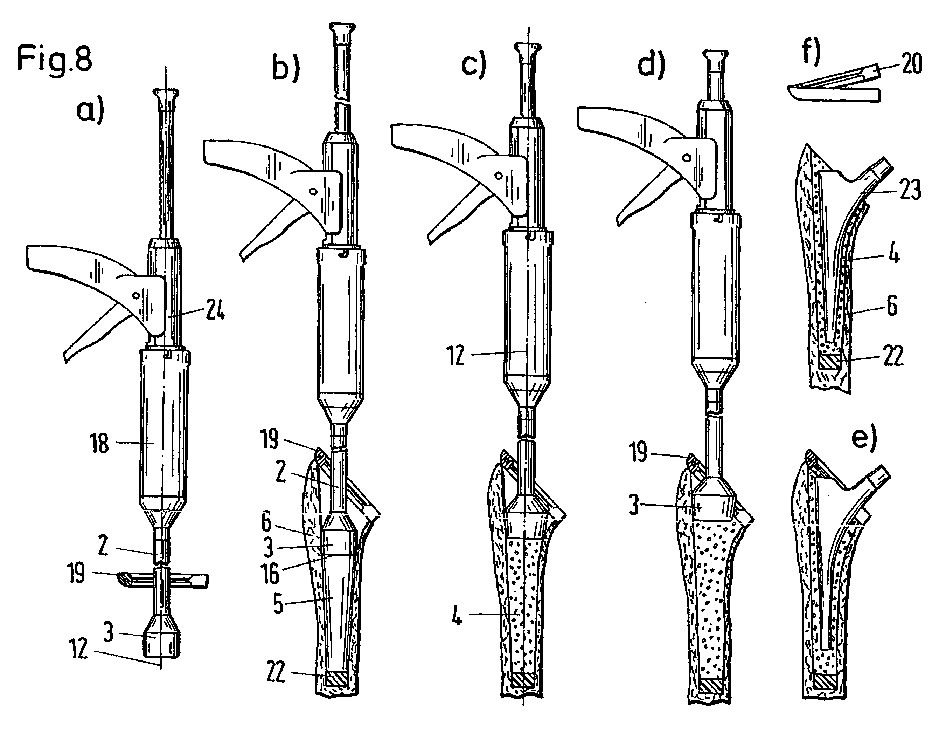

Die prinzipielle Anwendung der Einfüllvorrichtung 1 ist in den Figuren 8a bis f dargestellt. Fig. 8a zeigt eine montierte und mit Knochenzement gefüllte Einfüllvorrichtung mit Manschette 3, Einfüllkanüle 2, Siegel 19, Auspresszylinder 18 und Vorschubeinrichtung 24. In Fig. 8b ist das Siegel 19 auf einem vorbereiteten Femurknochen 6 bereits aufgesetzt und Einfüllkanüle 2 und Manschette 3 fahren in einen vorbereiteten intramedullären Kanal 5 ein bis die untere Kante 16 der Manschette an einer Markraumsperre 22 ansteht, deren Tiefe vorher gemessen wurde und mit einer Skala für die Eintauchtiefe auf der Einfüllkanüle vergleichbar ist. Durch Betätigen der Vorschubeinheit entsteht eine Situation wie sie im Detail zu Fig. 5 beschrieben wurde. Entsprechend dem ausgepressten Volumen an Knochenzement 4 wird die Einfüllvorrichtung in Fig. 8c nach oben geschoben. Der Operateur bestimmt dabei mit seiner Gegenkraft in der Richtung der Achse 12 wie gross der Druck auf dem Knochenzement beim Ablegen im intramedullären Kanal ist. In Fig. 8d ist die Manschette 3 bereits zur Hälfte im Siegel 19 geklemmt. Dies ist der Moment in dem die Einfüllvorrichtung um einen fiktiven Drehpunkt am Siegel 19 nach rechts geschwenkt wird, um den Raum unter dem Siegel vollständig mit Knochenzement zu füllen und die Manschette 3 vollständig herauszuziehen. Geführt durch das Siegel wird nun ein Femurprothesenschaft eingefahren bis dessen Endstellung wie in Fig. 8e erreicht ist. Dabei wird durch das Siegel ein Druck auf dem Knochenzement im intramedullären Kanal aufrechterhalten. Der überflüssige, am Siegel herausgepresste Knochenzement wird abgestreift. Nach dem Aushärten vom Knochenzement kann wie in Fig. 8f das Siegel als geschlitzter Ring 20 aufgespreizt werden und vom Prothesenschaft 23 entfernt werden. Zwischen Prothesenschaft 23 und Femurknochen 6 ist nun ein Zementmantel 4 ohne Hohlräume entstanden.

## Patentansprüche

1. Einfüllvorrichtung für das Einbringen von Knochenzement in den intramedullären Kanal (5) eines Knochens, vorzugsweise in den Kanal eines Femurknochens (6), mit einer Einfüllkanüle (2), an deren distalen Ende eine schlauchartige Manschette (3) befestigt ist, die unter dem Druck von austretenden Knochenzement (4) radial expandierbar ist, **dadurch gekennzeichnet, dass** der expandierbare Teil (7) der Manschette (3) im Längsschnitt radial eine über den Durchmesser und axial eine über eine unterste Austrittsöffnung (13, 13a) der Einfüllkanüle vorstehende Blechglockenform (9) derart hat, dass im unbelasteten Zustand der expandierbare Teil (7) der Manschette (3) mit zunehmendem Abstand von seiner Befestigung in seinem Querschnitt elliptisch (15) verformt und die untere Kante (16) der Manschette (3) nach innen eingezogen ist, um ein Hängenbleiben der Manschette (3) beim Absenken in den intramedullären Kanal (5) zu verhindern.

2. Einfüllvorrichtung nach Anspruch 1, **dadurch gekennzeichnet, dass** der untere Rand (10) der Manschette gegenüber einer tiefsten seitlichen Umlenkkante (11a) der untersten Austrittsöffnung (13, 13a) um einen Abstand 5 mm < a < 50 mm vorsteht, dass der bewegliche Teil der Manschette in einem Abstand. 3 mm < 1 < 50 mm über einer höchsten seitlichen Umlenkkante (11b) der Austrittsöffnungen (13, 13a) der Einfüllkanüle befestigt ist und dass der expandierbare Teil (7) der Manschette aus Gummi mit einer Dicke 1 mm < s < 2 mm besteht.

3. Einfüllvorrichtung nach einem der Ansprüche 1 oder 2, **dadurch gekennzeichnet, dass** die Austrittsöffnung (13) der Einfüllkanüle (2) in einer Ebene quer zur Achse (12) der Einfüllkanüle liegt.

4. Einfüllvorrichtung nach einem der Ansprüche 1 oder 2, **dadurch gekennzeichnet, dass** die Einfüllkanüle (2) durch einen Boden (14) abgeschlossen ist und über dem Boden seitliche Austrittsöffnungen (13a) aufweist.

5. Einfüllvorrichtung nach einem.der Ansprüche 1 bis 4, **dadurch gekennzeichnet, dass** die Dicke vom expandierbaren Teil (7) der Manschette s = 1,5 mm beträgt und dass die Manschette (3) eine Shore-Härte von 35A aufweist.

6. Einfüllvorrichtung nach Anspruch 3, **dadurch gekennzeichnet, dass** der Abstand a = 15 mm und der Abstand 1 = 20 mm betragen.

7. Einfüllvorrichtung nach Anspruch 4, **dadurch gekennzeichnet, dass** der Abstand a = 15 mm und der Abstand 1 = 5 mm betragen.

8. Einfüllvorrichtung nach einem der Ansprüche 1 bis 7, **dadurch gekennzeichnet; dass** sie eine Klemme (17) zum Befestigen der Manschette auf der Einfüllkanüle (2) aufweist.

9. Einfüllvorrichtung nach einem der Ansprüche 1 bis 8, **dadurch gekennzeichnet, dass** die Einfüllkanüle (2) mit einem Auspresszylinder (18) verbunden ist.

10. Einfüllvorrichtung nach Anspruch 9, **dadurch gekennzeichnet, dass** zwischen Auspresszylinder (18) und Manschette (2) ein Siegel (19) aufgesteckt ist, durch welches die Einfüllkanüle mit der Manschette rückwärts unter Seitenpressung ausziehbar ist.

11. Einfüllvorrichtung nach Anspruch 10, **dadurch gekennzeichnet, dass** das Siegel (19) aus einem geschlitzten Ring (20) aus Gummi besteht, welcher mit einem Bügel (21) verstärkt ist.

12. Einfüllvorrichtung nach einem der Ansprüche 10 oder 11, **dadurch gekennzeichnet, dass** das Siegel (19) transparent ist.

## Claims

1. Filling apparatus for the introduction of bone cement into the intramedullary cavity (5) of a bone, preferably into the cavity of a femur bone (6), comprising a filling cannula (2) at whose distal end a hose-like sleeve (3) is fastened which can be radially expanded under the pressure of emerging bone cement (4), **characterised in that**, in the longitudinal section, the expandable part (7) of the sleeve (3) has a sheet metal bell form (9) which projects radially beyond the diameter of the filling cannula and axially beyond a lowermost outlet opening (13, 13a) of the filling cannula, such that, in the unloaded state, the expandable part (7) of the sleeve (3) is elliptically deformed (15) in its cross-section with increasing distance from its attachment and the lower edge (16) of the sleeve (3) is drawn inwards in order to prevent the sleeve (3) being caught while being lowered into the intramedullary cavity (5).

2. Filling apparatus in accordance with claim 1 **characterised in that** the lower edge (10) of the sleeve projects by a distance 5 mm < a < 50 mm beyond a lowermost lateral deflection edge (11a) of the lowermost outlet opening (13, 13a); **in that** the movable part of the sleeve is fastened at a distance 3 mm < 1 < 50 mm above an uppermost lateral deflection edge (11b) of the outlet openings (13, 13a) of the filling cannula; and **in that** the expandable part (7) of the sleeve consists of rubber with a thickness 1 mm < s < 2 mm.

3. Filling apparatus in accordance with claim 1 or claim 2, **characterised in that** the outlet opening (13) of the filling cannula (2) lies in a plane transverse to the axis (12) of the filling cannula.

4. Filling apparatus in accordance with claim 1 or claim 2, **characterised in that** the filling cannula (2) is closed off by a base (14) and has lateral outlet openings (13a) above the base.

5. Filling apparatus in accordance with any one of the claims 1 to 4 **characterised in that** the thickness s of the expandable part (7) of the sleeve amounts to 1.5 mm; and **in that** the sleeve (3) has a Shore hardness of 35A.

6. Filling apparatus in accordance with claim 3 **characterised in that** the distance a amounts to 15 mm and the distance 1 amounts to 20 mm.

7. Filling apparatus in accordance with claim 4 **characterised in that** the distance a amounts to 15 mm and the distance 1 amounts to 5 mm.

8. Filling apparatus in accordance with any one of the claims 1 to 7, **characterised in that** it has a clamping clip (17) for fastening the sleeve to the filling cannula (2).

9. Filling apparatus in accordance with any one of the claims 1 to 8 **characterised in that** the filling cannula (2) is connected to an expulsion cylinder (18).

10. Filling apparatus in accordance with claim 9, **characterised in that** a seal (19) is pushed on between the expulsion cylinder (18) and the sleeve (2) through which the filling cannula with the sleeve can be withdrawn backwardly with pressure on the sides.

11. Filling apparatus in accordance with claim 10, **characterised in that** the seal (19) consists of a slit ring (20) of rubber which is reinforced by a wire hoop (21).

12. Filling apparatus in accordance with any one of the claims 10 or 11 **characterised in that** the seal (19) is transparent.

## Revendications

1. Dispositif de remplissage pour introduire du ciment à os dans le canal intramédulaire (5) d'un os, de préférence dans le canal d'un os de fémur (6), avec une canule d'introduction (2) à l'extrémité distale de laquelle est fixée une manchette tubulaire (3) qui, sous la pression du ciment à os (4) sortant, peut s'expanser radialement, **caractérisé en ce que** la partie apte à s'expanser (7) de la manchette (3) présente en coupe longitudinale une forme de cloche en tôle faisant saillie radialement sur le diamètre et axialement sur une ouverture de sortie la plus inférieure (13, 13a) de la canule de remplissage de telle sorte que, à l'état non chargé, la partie apte à s'expanser (7) de la manchette (3), au fur et à mesure que s'accroît l'écart relativement à sa fixation, est déformée dans sa section transversale d'une manière elliptique (15) et **en ce que** l'arête inférieure (16) de la manchette (3) est tirée vers l'intérieur pour empêcher un accrochage de la manchette (3) lors de l'abaissement dans le canal intramédulaire (5).

2. Dispositif de remplissage selon la revendication 1, **caractérisé en ce que** le bord inférieur (10) de la manchette dépasse par rapport à une arête de renvoi latérale la plus basse (11a) de l'ouverture de sortie la plus inférieure (13, 13a) d'une distance de 5 mm < a < 50 mm, **en ce que** la partie mobile de la manchette est fixée à un écart de 3 mm < 1 < 50 mm au-dessus d'une arête de renvoi latérale la plus haute (11b) des ouvertures de sortie (13, 13a) de la canule de remplissage, et **en ce que** la partie apte à s'expanser (7) de la manchette est constituée de caoutchouc d'une épaisseur de 1 mm < s < 2 mm.

3. Dispositif de remplissage selon l'une des revendications 1 ou 2, **caractérisé en ce que** l'ouverture de sortie (13) de la canule de remplissage (2) se situe dans un plan transversalement à l'axe (12) de la canule de remplissage.

4. Dispositif de remplissage selon l'une des revendications 1 ou 2, **caractérisé en ce que** la canule de remplissage (2) est fermée par un fond (14) et présente au-dessus du fond des ouvertures de sortie latérales (13a).

5. Dispositif de remplissage selon l'une des revendications 1 à 4, **caractérisé en ce que** l'épaisseur de 1a partie apte à s'expanser (7) de la manchette représente s = 1,5 mm et que la manchette (3) présente une dureté de Shore de 35A.

6. Dispositif de remplissage selon la revendication 3, **caractérisé en ce que** l'écart a = 15 mm et l'écart l = 20 mm.

7. Dispositif de remplissage selon la revendication 4, **caractérisé en ce que** l'écart a = 15 mm et l'écart l = 5 mm.

8. Dispositif de remplissage selon l'une des revendications 1 à 7, **caractérisé en ce qu'**il comporte une pince (17) pour fixer la manchette (7) sur la canule de remplissage (2).

9. Dispositif de remplissage selon l'une des revendications 1 à 8, **caractérisé en ce que** la canule de remplissage (2) est reliée à un cylindre d'expulsion (18).

10. Dispositif de remplissage selon la revendication 9, **caractérisé en ce qu'**il est mis en place entre le cylindre d'expulsion (18) et la manchette (2), une pièce de scellement (19) par laquelle la canule de remplissage peut être tirée en arrière avec la manchette sous pression latérale.

11. Dispositif de remplissage selon la revendication 10, **caractérisé en ce que** la pièce de scellement (19) est constituée d'une bague fendue (20) en caoutchouc qui est renforcée par un étrier (21).

12. Dispositif de remplissage selon l'une des revendications 10 ou 11, **caractérisé en ce que** la pièce de scellement (19) est transparente.
